(19) Europäisches Patentamt

European Patent Office

Office européen des brevets

(11) Numéro de publication : **0 340 698 B1**

# (12) FASCICULE DE BREVET EUROPEEN

(45) Date de publication du fascicule du brevet :
23.10.91 Bulletin 91/43

(21) Numéro de dépôt : **89107852.9**

(22) Date de dépôt : **29.04.89**

(30) Priorité : **03.05.88 CH 1651/88**

(43) Date de publication de la demande :
**08.11.89 Bulletin 89/45**

(45) Mention de la délivrance du brevet :
**23.10.91 Bulletin 91/43**

(84) Etats contractants désignés :
**AT BE CH DE ES FR GB IT LI NL SE**

(56) Documents cités :
**DE-A- 3 610 925**
**US-A- 4 681 102**

(51) Int. Cl.⁵ : **A61F 2/16**

(73) Titulaire : **ARCOFIL S.A.**
**Rue des Noyes 2**
**CH-2610 St-Imier (CH)**

(72) Inventeur : **Schenk, Bernard**
**Rue de la Citadelle 8**
**CH-2610 St-Imier (CH)**

(74) Mandataire : **Caron, Gérard**
**ICB Ingénieurs Conseils en Brevets SA**
**Passage Max. Meuron 6**
**CH-2001 Neuchâtel (CH)**

(54) **Instrument ophtalmologique.**

## Description

La présente invention concerne les instruments opthalmologiques, notamment ceux qui sont destinés à implanter un cristallin artificiel dans un oeil.

Une telle implantation est effectuée lorsque le cristallin naturel est atteint de cataracte. Depuis quelques années, la cataracte est traitée, en effet, en substituant un cristallin artificiel au cristallin naturel devenu opaque. Des cristallins artificiels en matière synthétique dure ont ainsi été implantés. A la longue, cette matière s'altère toutefois et perd progressivement sa transparence. De plus, l'implantation d'un tel cristallin implique nécessairement une incision relativement longue à la base de la cornée. Les patients doivent donc être hospitalisés pendant plusieurs jours après l'opération, pour laisser le temps à la plaie de se cicatriser.

Plus récemment, des cristallins artificiels en une autre matière synthétique ont été mis au point. La transparence de cette matière est pratiquement inaltérable. Cette dernière matière a, en outre, une souplesse remarquable. On peut la déformer considérablement ; aussitôt qu'on la relâche, elle reprend immédiatement sa forme originale.

Il serait ainsi possible d'introduire un cristallin en cette matière dans un oeil par une incision de trois millimètres de longueur au plus, qui, par ailleurs, permet d'éliminer le cristallin naturel, en même temps que l'humeur aqueuse sort de la chambre de l'oeil et de leur substituer un liquide physiologique pour la durée de l'opération.

Si la matière souple de l'implant comprenant ces cristallins artificiels n'adhère pas au support sur lequel elle repose et peut en être détachée sans peine en la soulevant, il est, en revanche, impossible de la faire glisser le long de ce support sans la détruire, en raison de son coefficient de frottement de glissement très élevé.

A l'aide d'une pince à trois griffes filiformes, dont deux sont engagées sous l'implant comprenant le cristallin artificiel et dont on presse la troisième, qui appuie au milieu du cristallin, au point de la faire passer entre les deux autres griffes, on enroule cet implant sur lui-même et peut l'introduire dans l'oeil par une incision de trois millimètres de longueur. Malheureusement, la pince ne peut pas être retirée seule de l'oeil, car ses griffes ne glissent pas le long de l'implant. Il faut par conséquent pratiquer une incision assez longue pour permettre à cette pince de sortir de l'oeil en position au moins partiellement ouverte.

Un instrument ophtalmologique permettant d'implanter un cristallin artificiel souple dans un oeil par une incision à la base de la cornée de l'ordre de 3 mm est décrit dans le brevet US-4681102. Cet instrument ophtalmologique comporte principalement un tube, un piston et un élément flexible et rigide pour enrouler sur lui-même un implant intraoculaire.

L'implant intraoculaire est tout d'abord placé sur l'élément flexible et rigide, qui est en position ouverte. Ce dernier est alors replié et introduit dans le tube de l'instrument ophtalmologique. L'implant souple se trouve alors enroulé à l'intérieur du tube.

Pour la mise en place de l'implant souple dans l'oeil, il suffit, après avoir introduit le tube dans l'oeil par une incision à la base de la cornée, de pousser l'implant souple à l'aide de la tige.

On constate donc que, lors de l'utilisation de cet instrument, l'implant souple est en contact avec le tube et doit glisser par rapport à celui-ci. Or, comme on l'a mentionné plus haut, la matière souple de l'implant présente un coefficient de frottement de glissement très élevé qui abîme irrémédiablement l'implant si on essaie de le faire glisser par rapport à un support.

Un instrument ophtalmologique pour un implant intraoculaire est également décrit dans le document DE-OS-3610925. Là encore, l'implant intraoculaire est directement en contact avec le tube lorsqu'il est repoussé hors de celui-ci.

La présente invention a pour but un instrument ophtalmologique qui permette d'implanter un cristallin artificiel souple dans un oeil par une toute petite incision à la base de la cornée au point d'en arriver à un traitement ambulatoire de la cataracte, et qui soit dépourvue notamment des inconvénients mentionnés ci-dessus.

L'instrument selon l'invention comprend un guide tubulaire dans lequel peut coulisser un piston, celui-ci pouvant être manipulé par son extrémité postérieure et comportant, du côté de son extrémité antérieure, un film mince, souple, dans lequel un implant intra-oculaire souple comprenant un cristallin artificiel peut être enroulé de manière que ledit film entoure l'implant.

Le film souple peut être fixé de n'importe quelle manière à l'extrémité antérieure du piston, notamment par collage, soudage, sertissage ou autre. Il peut également être simplement pincé entre deux branches élastiques prévues sur l'extrémité antérieure du piston. Ce film mince peut être réalisé en une partie ou en plusieurs parties, notamment en fonction de sa rigidité ou de l'épaisseur de l'implant intra-oculaire.

On comprend que le film mince enveloppe l'implant intra-oculaire lorsqu'il est introduit dans le guide, par déplacement du piston, ce qui supprime le frottement de glissement entre l'implant intra-oculaire et la paroi interne du guide.

Pour faciliter le repliement de l'implant intra-oculaire et son introduction dans le guide, l'extrémité antérieure de ce dernier peut être avantageusement munie d'un embout en forme d'entonnoir. Celui-ci sert uniquement à la mise en place de l'implant intra-oculaire dans le guide et peut être ensuite retiré.

Les caractéristiques et avantages de l'invention ressortiront mieux de la description qui va suivre, donnée à titre illustratif mais non limitatif, en référence aux dessins annexés, sur lesquels :

— la figure 1 est une coupe diamétrale selon un plan vertical d'un instrument ophtalmologique selon l'invention, sur lequel est déposé un implant intra-oculaire souple ;

— la figure 2 est une coupe semblable à celle de la figure 1, après une première phase de manipulation de l'instrument ;

— la figure 3 est une vue en plan, dans la position de la figure 1, une partie étant coupée selon un plan diamétral horizontal ;

— la figure 4 est une coupe selon la ligne IV-IV de la figure 1 ;

— la figure 5 est une coupe selon la ligne V-V de la figure 2 ;

— la figure 6 est une coupe transversale de l'instrument, prêt à introduire un implant souple, comprenant un cristallin artificiel, dans un œil ;

— les figures 7, 8 et 9 illustrent différentes variantes de réalisation du film souple ;

— la figure 10 est une coupe diamétrale selon un plan vertical d'un instrument ophtalmologique selon un autre mode de réalisation de l'invention;

— la figure 11 est une vue en plan dans la position de la figure 10, une partie étant coupée selon un plan diamétral horizontal ;

— la figure 12 est une coupe selon la ligne XII-XII de la figure 10 ;

— la figure 13 est une coupe selon la ligne XIII-XIII de la figure 11 ; et

— la figure 14 est une coupe transversale de l'instrument, prêt à introduire un implant souple, comprenant un cristallin artificiel, dans un œil.

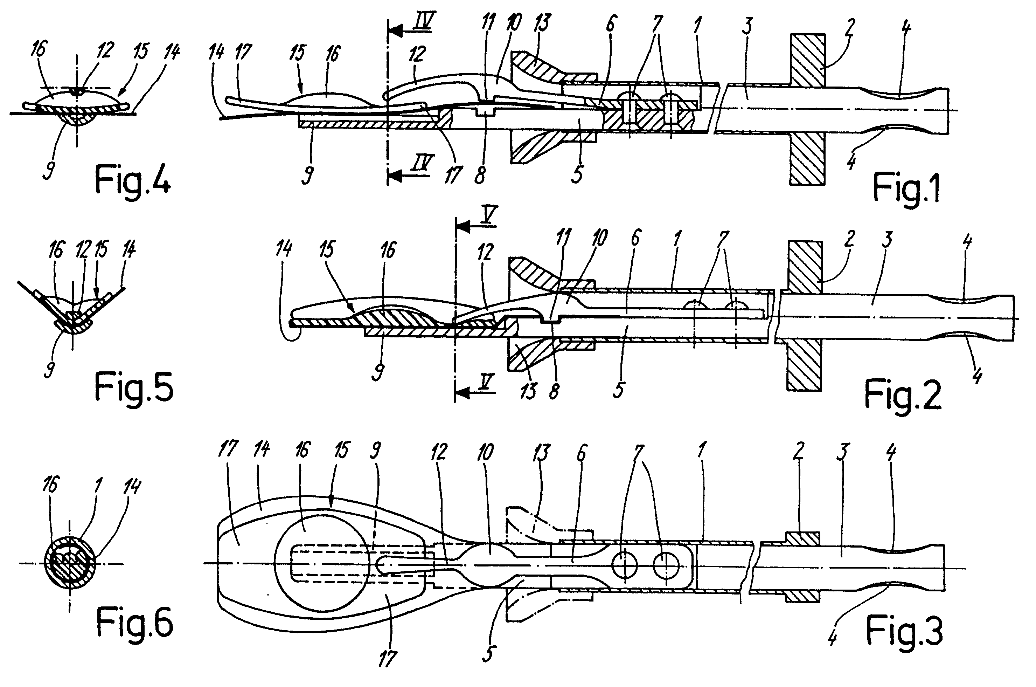

L'instrument représenté sur les figures 1 à 6 comprend un tube 1, qui est représenté avec un profil circulaire (figure 6). Pour des raisons exposées ci-après, ce tube aura cependant de préférence une forme aplatie, par exemple ovale. A son extrémité postérieure, le tube 1 présente un barreau 2 qui en est solidaire, pour en permettre la manipulation. Un piston 3 est monté coulissant à frottement doux dans le tube 1. Des encoches 4 sont formées à l'extrémité postérieure du piston 3, pour en faciliter la préhension. A son extrémité antérieure, le piston 3 présente une pince à deux branches 5, 6. La branche 5 est venue de fabrication en une pièce avec le piston 3. Quant à la branche 6, elle est fixée à la branche 5 par des rivets 7 ; elle est élastique et tend normalement à s'écarter de la branche 5.

Dans l'exemple représenté, les pièces décrites de l'instrument sont métalliques. De préférence, elles seront cependant faites en une matière synthétique. Dans ce cas, la branche 6 sera fixée sur la branche 5 par soudage à ultrasons ou par collage.

La face supérieure de la branche 5 présente une encoche 8. L'extrémité 9 de la branche 5 a un profil en arc de cercle, dont la concavité est dirigée vers le haut. Vers son extrémité, la branche 6 présente un renflement 10 et un ergot 11 dans sa face inférieure. Le renflement 10 se termine par un nez étroit 12, arqué vers le bas. Ce dernier, plus étroit que l'extrémité 9 de la branche 5, s'étend au-dessus de celle-ci sans la toucher, en position de repos de l'instrument (figure 1). Dans cette position, un embout 13, en forme d'entonnoir est glissé sur l'extrémité antérieure du tube 1.

Un film souple 14, très mince (deux à trois centièmes de millimètre d'épaisseur) est encore disposé sur la branche 5. Dans le cas d'un instrument non-jetable, tel que représenté sur les figures 1 à 6, un nouveau film souple 14 est simplement posé sur la branche 5 avant chaque implantation. Si, en dépit de sa minceur, le film 14 devait être encore trop rigide, une ou plusieurs fentes pourraient être formées dans ce film, dans le sens de sa longueur.

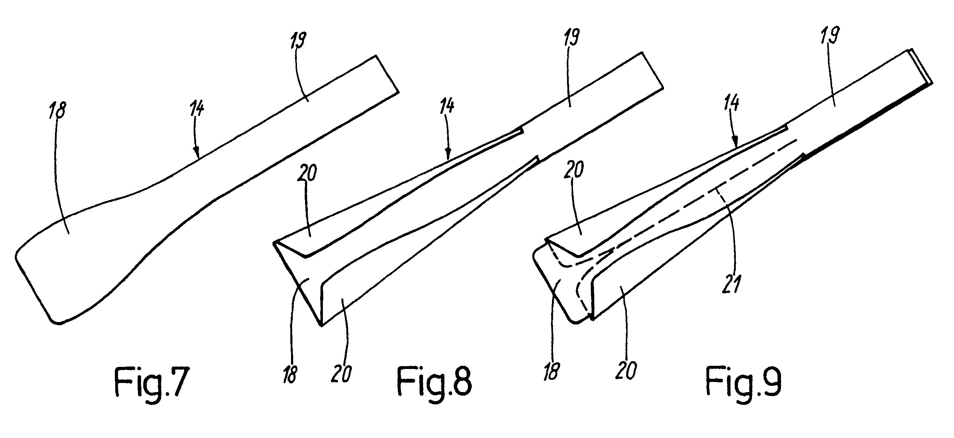

Différentes variantes de réalisation de film souple 14 sont représentées sur les figures 7, 8 et 9. Le film souple 14 peut être constitué d'une feuille unique découpée pour former un réceptacle 18, destiné à recevoir un implant intra-oculaire, et une languette 19 destinée à être fixée de manière amovible ou non au piston 3 (figure 7). Le film souple 14 peut aussi comprendre un réceptacle 18 plus large dont les côtés sont repliés sur le dessus du réceptacle pour former des bords 20. Ceux-ci permettent de mieux maintenir un implant intra-oculaire sur le réceptacle 18 et facilite l'enroulement de l'implant (figure 8). Les bords 20 peuvent également être réalisés dans une seconde feuille disposée sous la feuille dans laquelle est formé le réceptacle (figure 9). Des fentes, telles que 21, peuvent être prévues pour donner plus de souplesse au film 14 et pour faciliter l'enlèvement du film 14 de l'oeil après la mise en place de l'implant intra-oculaire, en divisant le film souple 14 en plusieurs parties d'un coup de ciseaux.

L'instrument décrit est prêt à être utilisé. A cet effet, un implant intra-oculaire souple 15 est disposé sur le film 14, lui tenant lieu de réceptacle. L'implant 15 comprend un cristallin artificiel 16 venu en une pièce avec deux ailes 17 pour centrer le cristallin 16 dans l'oeil. Le cristallin 16 est naturellement choisi en fonction de la morphologie de l'oeil à traiter et de manière à en corriger les anomalies dans la mesure du possible.

En premier lieu, le piston 3 est retiré vers l'arrière du tube 1. Lorsque le renflement 10 commence à entrer dans le tube 1, il pousse la branche 6 contre la branche 5. L'ergot 11 entre alors dans l'encoche 8, ce qui solidarise le film 14 à la pince 5, 6 et par conséquent au piston 3. Simultanément, l'extrémité du nez 12 descend sur l'une des ailes 17 de l'implant 15. Ce nez 12 presse ainsi l'aile 17 ainsi que le film 14 au fond de la concavité de l'extrémité 9 de la branche 5 (figure 5). Le film 14 prend une forme en V et l'implant épouse aussi cette forme.

En continuant à retirer le piston 3 du tube 1, le film 14 et l'implant 15 viennent en contact avec l'embout 13. Celui-ci rapproche progressivement l'un de l'autre les bords opposés du film 14 jusqu'au point de les rabattre l'un sur l'autre en enroulant l'implant 15 sur lui-même et en faisant entrer le film 14 et l'implant 15 dans le tube 1 (figure 6). Comme seul le film 14 entre en contact d'abord avec l'embout 13 puis avec le tube 1, il glisse sans difficulté dans ce tube.

L'embout 13 est alors retiré du tube 1, dont l'extrémité peut être introduite dans l'incision pratiquée à la base de la cornée. Au préalable, le cristallin naturel a évidemment été éliminé par cette incision, de même que l'humeur aqueuse de l'oeil et un liquide physiologique leur a été substitué. A ce stage de l'opération, on remarquera qu'un tube aplati sollicite les parois de l'incision moins fortement qu'un tube de profil circulaire. Pour introduire l'extrémité du tube 1 dans une incision de trois millimètres de longueur, le grand diamètre du tube 1 doit, bien sûr, être un peu plus faible.

L'implant 15 est introduit dans la chambre antérieure de l'oeil simplement en poussant le piston 3 dans le tube 1, de la même manière qu'on procède avec les seringues hypodermiques. Le film 14, évidemment choisi avec un faible coefficient de frottement de glissement, glisse sans peine hors du tube. Dès que l'implant 15 entre dans la chambre antérieure de l'oeil, il reprend sa forme originale, représentée aux figures 1, 3 et 4. Quand il est entré entièrement dans cette chambre, il flotte pratiquement sur le film 14, dans le liquide physiologique qui a été injecté dans l'oeil ; il n'est donc plus pressé contre le film 14, qui peut être retiré seul de l'oeil.

Il suffit alors de faire passer l'implant 15 à travers la pupille, dans la chambre postérieure de l'oeil, puis d'injecter une nouvelle humeur aqueuse dans l'oeil en refoulant le liquide physiologique à travers l'incision. Un seul point de suture suffit à refermer une incision de trois millimètres pratiquée à la base de la cornée, de sorte qu'une hospitalisation subséquente du patient n'est pas nécessaire.

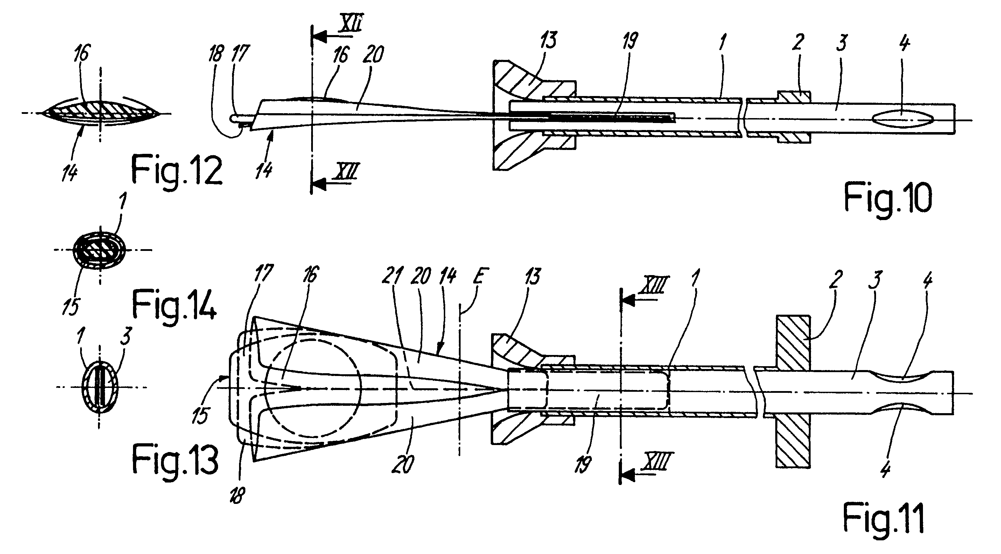

On a représenté sur les figures 10 à 14 une version jetable de l'instrument ophtalmologique selon l'invention. Sur ces figures, les éléments identiques à ceux de l'instrument non-jetable représenté sur les figures 1 à 6 portent les mêmes références.

L'instrument jetable comprend un tube 1, à section ovale, sur l'extrémité postérieure duquel est prévu un barreau 2 pour en permettre la manipulation. Un piston 3 est monté coulissant à frottement doux dans le tube. Ce piston est pourvu d'encoches 4 à proximité de son extrémité postérieure, pour en faciliter la préhension. Le film souple 14 est fixé à l'extrémité antérieure du piston 3. Ceci peut être réalisé par collage, soudage, sertissage ou autre. Le piston 3 et le film souple 14 pourraient également être faits en une seule pièce, par injection. Un embout 13 est monté sur l'extrémité antérieure du tube 1 pour faciliter l'introduction de l'implant 15 dans ce tube. L'embout 13 est ensuite retiré avant l'introduction du tube dans l'oeil.

L'utilisation de l'instrument jetable représenté sur les figures 10 à 14 est identique à celle de l'instrument non-jetable représenté sur les figures 1 à 6. Cependant, le film souple 14 peut être séparé en plusieurs parties, après la mise en place de l'implant 15, par un coup de ciseaux suivant la ligne E (figure 11). On peut alors extraire plus facilement les différentes parties du film souple 14 que si celui-ci était en une seule partie dont les bords et le réceptacle étaient en contact avec l'implant 15.

## Revendications

1. Instrument ophtalmologique destiné à implanter un cristallin artificiel dans un oeil comprenant un guide tubulaire (1) dans lequel est monté un piston coulissant (3), caractérisé en ce qu'il comprend un film mince souple (14) à l'extrémité antérieure du piston, dans lequel un implant intra-oculaire souple (15), comprenant le cristallin artificiel (16), peut être enroulé de manière que ledit film entoure l'implant.

2. Instrument selon la revendication 1, caractérisé en ce que le film (14) comprend un réceptacle (18) pour l'implant et deux bords repliés (20) destinés à recouvrir partiellement l'implant.

3. Instrument selon l'une quelconque des revendications 1 et 2, caractérisé en ce que le film (14) comprend au moins une fente (21) longitudinale.

4. Instrument selon l'une quelconque des revendications 1 à 3, caractérisé en ce que l'extrémité antérieure du piston est muni d'une pince (5, 6) pour maintenir le film (14).

5. Instrument selon la revendication 4, caractérisé en ce que l'une des branches (6) de la pince est élastique, ladite pince étant normalement ouverte lorsqu'elle est hors du guide tubulaire.

6. Instrument selon l'une quelconque des revendications 1 à 3, caractérisé en ce que le piston (3) et le film (14) sont faits en une seule pièce, par injection.

7. Instrument selon l'une quelconque des revendications 1 à 6, caractérisé en ce que le guide tubulaire (1) est à section ovale.

## Claims

1. Ophthalmological instrument intended for the implantation of an artificial crystalline lens into an eye comprising a tubular guide (1) in which is mounted a sliding piston (3), characterized in that it comprises a flexible thin film (14) at the front end of the piston in which a flexible intraocular implant (15) comprising the artificial crystalline lens (16) may be wrapped in a manner such that the film surrounds the implant.

2. Instrument according to claim 1, characterized in that the film (14) comprises a receptacle (18) for the implant and two folded back edges (20) intended to cover the implant partially.

3. Instrument according to any one of claims 1 and 2, characterized in that the film (14) includes at least one longitudinal slit (21).

4. Instrument according to any one of claims 1 to 3, characterized in that the front end of the piston is provided with a clip (5, 6) for holding the film (14).

5. Instrument according to claim 4, characterized in that one of the arms (6) of the clip is elastic, said clip being normally open when outside the tubular guide.

6. Instrument according to any one of claims 1 to 3, characterized in that the piston (3) and the film (14) are integrally fashioned in a single piece by injection.

7. Instrument according to any one of claims 1 to 6, characterized in that the tubular guide (1) is of oval cross-section.


## Patentansprüche

1. Ophtalmologisches Instrument, bestimmt zum Implantieren einer künstlichen Linse in ein Auge, mit einer rohrförmigen Führung (1), in der ein Gleitkolben (3) angeordnet ist, dadurch gekennzeichnet, daß es eine dünne weiche Folie (14) am vorderen Ende des Kolbens umfaßt, in die ein intraokulares weiches Implantat (15) mit einer künstlichen Linse (16) derart einhüllbar ist, daß die Folie das Implantat umgibt.

2. Instrument nach Anspruch 1, dadurch gekennzeichnet, daß die Folie (14) eine Aufnahme (18) für das Implantat und zwei zurückgeschlagene Ränder (20), bestimmt zum teilweisen Abdecken des Implantats, aufweist.

3. Instrument nach einem der Ansprüche 1 und 2, dadurch gekennzeichnet, daß die Folie (14) mindestens einen Längsschlitz (21) aufweist.

4. Instrument nach einem der Ansprüche 1 bis 3, dadurch gekennzeichnet, daß das vordere Ende des Kolbens mit einem Greifer (5, 6) zum Halten der Folie (14) versehen ist.

5. Instrument nach Anspruch 4, dadurch gekennzeichnet, daß einer der Greiferarme (6) elastisch ist, wobei der Greifer normalerweise, wenn er sich außerhalb der rohrförmigen Führung befindet, offen ist.

6. Instrument nach einem der Ansprüche 1 bis 3, dadurch gekennzeichnet, daß der Kolben (3) und die Folie (14) einstückig mittels Spritzguß gefertigt sind.

7. Instrument nach einem der Ansprüche 1 bis 6, dadurch gekennzeichnet, daß die rohrförmige Führung (1) von ovalem Querschnitt ist.

Fig.4

Fig.5

Fig.6

Fig.1

Fig.2

Fig.3

EP 0 340 698 B1

Fig.7  Fig.8  Fig.9

EP 0 340 698 B1

Fig.12

Fig.10

Fig.14

Fig.13

Fig.11

EP 0 340 698 B1